# EUROPEAN PATENT SPECIFICATION

(11) **EP 3 042 650 B1**
(45) Date of publication and mention of the grant of the patent: **10.06.2020**
(21) Application number: 16158309.1
(22) Date of filing: 27.03.2014
(51) Int. Cl.: A61K 31/00, A61K 31/5377, A61K 31/554, A61K 31/7068, A61P 35/00

(54) **BROMODOMAIN INHIBITOR JQ1 IN COMBINATION WITH DNA METHYLATION INHIBITOR DAC FOR USE IN CANCER TREATMENT**
BROMDOMÄNENHEMMER JQ1 IN KOMBINATION MIT DNA-METHYLIERUNGSHEMMER DAC ZUR VERWENDUNG BEI DER KREBSBEHANDLUNG
COMBINAISON DE JQ1, UN INHIBITEUR DE BROMODOMAIN AVEC L'INHIBITEUR DE LA MÉTHYLATION D'ADN DAC POUR LE TRAITEMENT DU CANCER

(43) Date of publication of application: 13.07.2016
(62) Divisional of application: 14161897.5
(73) Proprietor: Palacky University, Olomouc, 77147 Olomouc (CZ)
(72) Inventor: AGRAWAL, Khushboo, 77900 Olomouc (CZ); DZUBAK, Petr, 75103 Brodek u Prerova (CZ); FRYDRYCH, Ivo, 75114 Drevohostice (CZ); HAJDUCH, Marian, 79305 Moravsky Beroun (CZ)
(74) Representative: Hartvichova, Katerina

(56) References cited:
- WO-A2-03/065995
- RONALD X XU ET AL: "Drug-loaded biodegradable microspheres for image-guided combinatory epigenetic therapy in cells", JOURNAL OF BIOMEDICAL OPTICS 2010 SEP-OCT LNKD- PUBMED:21054101, vol. 16, no. 2, 1 February 2011 (2011-02-01), pages 20507-1, XP055277486, ISSN: 1560-2281, DOI: 10.1117/1.3548878
- M. BOI ET AL: "The BET Bromodomain Inhibitor OTX015 Affects Pathogenetic Pathways in Preclinical B-cell Tumor Models and Synergizes with Targeted Drugs", CLINICAL CANCER RESEARCH, vol. 21, no. 7, 26 January 2015 (2015-01-26), pages 1628-1638, XP055227481, US ISSN: 1078-0432, DOI: 10.1158/1078-0432.CCR-14-1561
- Marie-Magdelaine Coudé ET AL: "BET inhibitor OTX015 targets BRD2 and BRD4 and decreases c-MYC in acute leukemia cells", Oncotarget, 10 July 2015 (2015-07-10), page 17698, XP055213103, United States Retrieved from the Internet: URL:http://www.ncbi.nlm.nih.gov/pubmed/259 89842 [retrieved on 2016-06-03]
- AGRAWAL KHUSHBOO ET AL: "5-azacytidine nucleosides and their derivatives: Molecular hallmarks of drug resistance & alternative therapeutic regimen", CANCER RESEARCH, vol. 75, no. Suppl. 15, August 2015 (2015-08), page 2944, XP8180500, & 106TH ANNUAL MEETING OF THE AMERICAN-ASSOCIATION-FOR-CANCER-RESEARCH (AACR); PHILADELPHIA, PA, USA; APRIL 18 -22, 2015
- ASTORGUES-XERRI LUCILE ET AL: "In vitro evaluation of OTX015, a novel pan-BET-bromodomain (BET-BRD) inhibitor, as single agent and in combination with standard chemotherapy drugs in human leukemic cell lines", CANCER RESEARCH, vol. 74, no. 19, Suppl. S, October 2014 (2014-10), page 5529, XP8180499, & 105TH ANNUAL MEETING OF THE AMERICAN-ASSOCIATION-FOR-CANCER-RESEARCH (AACR); SAN DIEGO, CA, USA; APRIL 05 -09, 2014

## Description

### Field of Art

The invention is directed to a bromodomain inhibitor in combination with a DNA methylation inhibitor for use in treatment of cancers, in order to overcome resistance towards DNA methylation inhibitors.

### Background Art

Resistance to chemotherapeutic treatment is one of the major impediments forefending the successful cancer therapy (Gottesman M.M. et al., Nature Reviews Cancer 2002; 2, 48-58). Although the research has unraveled the main molecular signatures of resistance to chemotherapy, including intracellular inactivation of the drug (Garattini S. et al., European Journal of Cancer 2007; 43, 271-82), defects in DNA mismatch repair (Fink D. et al., Clinical Cancer Research 1998; 4, 1-6), evasion of apoptosis (Hanahan D. et al., Cell 2000; 100, 57-70), membrane transporters (Huang Y. et al., Cancer Research 2004; 64, 4294-301) and many more, the failure of cancer chemotherapy remains frequently unresolved. Moreover, a particular drug resistance mechanism defined in cell culture systems and animal models does not necessarily correlate with the individual molecular pathology in clinic (Cimoli G. et al., Biochimica et Biophysica Acta 2004; 1705, 103-20). This has underlined the paramount importance for investigating the additional targets to sensitize the cancer patients, resistant to a particular drug, and tailor an alternative therapeutic regimen for the individual patients. Currently, epigenetics has emerged as one of the most promising fields expanding the boundaries of oncology and aberrant DNA methylation remains the consistent hallmark due to its frequent involvement in all types of cancer (Rodríguez-Paredes M. et al., Nature Medicine 2011; 17, 330-39). Cytosine analogues, 5-azacytidine (azacytidine) and 2'-deoxy-5-azacytidine (decitabine) are currently one of the most effective epigenetic drugs (Stresemann C. et al., International Journal of Cancer 2008; 123, 8-13), which function by inhibiting the expression of *de novo* DNA methyltransferases and have shown substantial potency in reactivating tumor suppressor genes silenced by aberrant DNA methylation (Karahoca M. et al., Clinical Epigenetics 2013; 5, 3). The prototypical DNA methyltransferase inhibitors, azacytidine and decitabine are one of the few drugs that patients suffering from myelodysplastic syndromes (MDS) and acute myeloid leukemia (AML) respond to, and have been approved by the Food And Drug Administration (FDA) and European Medicines Agency (EMA) for the treatment of MDS (Saba H.I. et al., Therapeutics and Clinical Risk Management 2007; 3, 807-17). Apart from being established therapies for myeloid malignancies, they seemed promising in eradicating solid tumors during early clinical trials (Cowan L.A. et al., Epigenomics 2010; 2, 71-86). However, like other anti-cancer drugs, resistance to these hypomethylating agents is the major barrier, reversing the effective epigenetic therapy. Most patients do not respond to therapy and experience primary resistance whereas those responding initially acquire secondary resistance and succumb to the disease, despite continued therapy (Prébet T. et al., Journal of Clinical Oncology 2011; 29, 3322-7). Molecular mechanisms dilating the cause of resistance to these drugs *in vitro* are diverse, including insufficient drug influx by membrane transporters, deficiency of the enzyme deoxycytidine kinase required for drug activation, or deamination by cytidine deaminase leading to increased drug metabolism, but they fail to explain acquired resistance in patients. In addition, it has also been implemented that the secondary resistance to decitabine is likely to be independent of DNA methylation and the resistance develops regardless of persistent demethylation (Qin T. et al., PLOS ONE 2011; 6, e23372). Also, it is undeniable fact that the re-expression of epigenetically silenced tumor suppressor genes following decitabine treatment is transitory (Kagey J.D. et al., Molecular Cancer Research 2010; 8, 1048-59). Withdrawal of decitabine eventually results in gene re-silencing leading to resistance whereas sustained gene re-expression concords with the clinical response, supporting the role of gene re-silencing in development of drug resistance (Hesson L.B. et al., PLOS Genetics 2013; 9, e1003636). If the focus is laid on gene re-silencing as the prerequisite for resistance, it highlights the central dogma of epigenetics which articulates that the gene silencing mechanisms (DNA hypermethylation, mutations in chromatin remodeling complexes and multiple post-translational histone modifications) are not isolated from each other but interlinked (Grant S. et al., Clinical Cancer Research 2009; 15, 7111-3). In this context, bromodomains (BRDs), chromatin effector modules that recognize and bind to ε-N-acetyl lysine motifs have rapidly emerged as exciting new targets in the quest for clinical progress in cancer (Muller S. et al., Expert Reviews in Molecular Medicine). The role of multiple bromodomain genes in restricting the spread of heterochromatic silencing has been explored in the past (Jambunathan N. et al., Genetics 2005; 171, 913-22). In addition, bromodomain proteins play a critical role in gene activation by recruitment of the factors necessary for transcription (Josling G.A. et al., Genes 2012; 3, 320-43). Human BRD family comprises 47 bromodomain containing genes and/or proteins including the bromodomain and extra terminal domain (BET) family composed of BRD2, BRD3, BRD4, and BRDT (Puissant A. et al., Cancer Discovery 2012; 3, 1-16).
The present invention provides alternative therapeutic regimens to resolve the resistance towards DNA methylation inhibitors.

### Disclosure of the invention

The present invention provides bromodomain inhibitor in combination with DNA methylation inhibitor for use in DNA methylation inhibitor therapy of cancer.

The DNA methylation inhibitor is 2'-deoxy-5-azacytidine (DAC).

The selective BET bromodomain inhibitor is (+)-JQ1.

The decrease in the IC₅₀ values of the tested BET bromodomain inhibitor was determined repeatedly in several drug resistant cell lines in comparison with their genetically identical drug sensitive counterpart, which indicates towards the sensitivity of the DAC resistant cells to BET bromodomain inhibitor. Therefore, bromodomain inhibitor (+)-JQ1 can be used in combination with the DNA methylation inhibitor to re-sensitize the patients, resistant to a DNA methylation inhibitor.

The patients who initially respond to the DNA methylation inhibitor but during prolonged treatment develop the sign of disease progression by acquiring secondary resistance to the drug can be re-sensitized by the use of the bromodomain inhibitor in combination with the DNA methylation inhibitor. This provides the alternative therapeutic regimen to overcome the resistance and may reduce the incidence of developing resistance to the DNA methylation inhibitor.

The combination therapy is particularly useful in cancers selected from carcinomas, sarcomas, melanomas, lymphomas, and leukemia.

### Examples of carrying out the invention

### Cell culture/Development of resistant cell lines

We used the cell line derived from the patient suffering from human colorectal cancer, HCT116 (American Type Culture Collection, Manassas, VA, USA) to study the mechanism of resistance towards DNA methylation inhibitor, 2'-deoxy-5-azacytidine. Cell line was maintained in McCoy's 5A medium (Sigma-Aldrich, St. Louis, MO) supplemented with 10% fetal bovine serum (PAN-Biotech GmbH, Aidenbach, Germany), 100 U/mL penicillin (Biotika, Slovenská L'upča, Slovak Republic), 50 µg/mL streptomycin and 3 mM L-glutamine (Sigma-Aldrich) at 37°C and 5% CO₂.
The resistant cell lines were developed using two methods. For the purpose, early passage HCT116 cells were split and seeded into two Petri dishes. The cells in Petri dish A were initially treated with 1x IC₅₀ concentration of the drug (0.28 µM) which was gradually increased with the adaptation of resistance up to 10x IC₅₀ in subsequent passages. The cells in Petri dish B were directly exposed to 5x IC₅₀ concentration of the drug which was further doubled to 10x IC₅₀. After long term exposure of the cells to cytotoxic dose of the drug, the bulk population was determined to be resistant. Cloning of the resistant cell population resulted in six resistant cell lines, R1.1, R1.2, R1.3, R1.4 (isolated from Petri dish A) and R2.1, R2.2 (isolated from Petri dish B).
Resistance was confirmed by the MTT-based cell survival assay and the resistance index was calculated as the fold increase in the IC₅₀ of the resistant cell lines compared with the untreated control. All of the resistant cell lines were >100 folds resistant to DAC.

### Determination of cross resistance/ sensitivity

MTT based cell survival assay is primarily based on the reduction of the yellow colored tetrazolium salt, 3-(4,5-dimethylthiazol-2-yl)-2,5-diphenyltetrazolium bromide (MTT) to the insoluble purple colored formazan crystals by NAD(P)H-dependent oxidoreductase enzymes in the mitochondria of the viable cell. The intensity of the purple color produced on solubilization of the formazan crystals is directly proportional to the number of viable cells (Meerloo J.V. et al., Methods in Molecular Biology 2011; 731, 237-45).
To determine the half maximal inhibitory concentration (IC₅₀) of the drugs, DAC, ALPHA DAC, (+)-JQ1 (Tocris Bioscience, Bristol, United Kingdom), four independent experiments were performed using triplicate wells of 96 well plates. Cells in 80 µL of medium were plated in each of the experimental and the control wells followed by addition of 20 µL of medium with five-fold concentration of the drug to experimental wells. The wells with medium alone were included alongside to provide the blank for absorbance readings. After 72 h of treatment with drug, 10 µL of MTT (Sigma-Aldrich) prepared in 1x PBS (5 mg/mL) was added to all wells including blank and control and incubated until the purple formazan crystals were visible after which 100 µL of the detergent (10% SDS, pH: 5.5) was added and the plates were incubated overnight to solubilize the formazan and the cellular material. MTT absorbance was read at 540 nM using Labsystems iEMS Reader MF, and the IC₅₀ values were determined using the Chemorezist software (IMTM, Palacky University, Olomouc, Czech Republic).
Resistance was determined as the fold increase, whereas, the sensitivity was determined as the fold decrease, in the IC₅₀ of the drugs for the resistant cell lines compared to their genetically identical drug sensitive counterpart.

### Results

Resistance towards DAC and ALPHA-DAC was determined using MTT based cell survival assay and the resistance index was calculated as the ratio of the IC₅₀ values of the resistant cell lines to their genetically identical drug sensitive counterpart.
The values in the table represents mean IC₅₀ in µM calculated from four independent experiments, each performed in triplicates (S.D, ±0 - ±7) and the values in parentheses represents fold changes. The experimental data is statistically significant (**p<0.005, ***p<0.0005).

| **Cell lines** | **Mean IC₅₀ values in µM (fold changes)** | |
|---|---|---|
| | **DAC** | **ALPHA DAC** |
| HCT116 | 0.28 | 2.16 |
| HCT116-DAC (R1.1) | >100 (>357) *** | >100 (>46.3) *** |
| HCT116-DAC (R1.2) | >100 (>357) *** | >100 (>46.3) *** |
| HCT116-DAC (R1.3) | >100 (>357) *** | >100 (>46.3) *** |
| HCT116-DAC (R1.4) | >100 (>357) *** | >100 (>46.3) *** |
| HCT116-DAC (R2.1) | >100 (>357) *** | >100 (>46.3) *** |
| HCT116-DAC (R2.2) | >100 (>357) *** | >100 (>46.3) *** |

Sensitivity towards bromodomain inhibitor was determined using MTT based cell survival assay and the sensitivity index was calculated as the ratio of the IC₅₀ values of the parental cell line to their genetically identical drug resistant counterpart or the resistant cell lines. The values in the table represents mean IC₅₀ in µM calculated from four independent experiments, each performed in triplicates (S.D, ±0.05 - ±1.89) and the values in parentheses represents fold changes. The experimental data is statistically significant, (*p<0.05, **p<0.005, ***p<0.0005).

| **Cell lines** | **Mean** IC₅₀ **values in µM (fold changes)** |
|---|---|
| | **(+)-JQ1** |
| HCT116 | 3.06 |
| HCT116-DAC (R1.1) | 0.67 (-4.57) *** |
| HCT116-DAC (R1.2) | 0.99 (-3.11) ** |
| HCT116-DAC (R1.3) | 0.44 (-6.97) *** |
| HCT116-DAC (R1.4) | 0.65 (-4.71) *** |
| HCT116-DAC (R2.1) | 1.29 (-2.38) ** |
| HCT116-DAC (R2.2) | 0.61 (-5.05) *** |

## Claims

1. Bromodomain inhibitor (+)-JQ1 in combination with DNA methylation inhibitor 2'-deoxy-5-azacytidine for use in DNA methylation inhibitor therapy of cancer to overcome the resistance and/or to reduce the incidence of developing resistance to DNA methylation inhibitor 2'-deoxy-5-azacytidine.

2. Bromodomain inhibitor in combination with DNA methylation inhibitor for use according to claim 1, wherein the cancer is selected from carcinomas, sarcomas, melanomas, lymphomas, and leukemia.

## Patentansprüche

1. Bromodomäneninhibitor (+)-JQ1 in Kombination mit dem DNA-Methylierungsinhibitor 2'-Desoxy-5-azacytidin zur Verwendung in der DNA-Methylierungsinhibitor-Therapie von Krebs, um die Resistenz zu überwinden und/oder das Auftreten einer Resistenzentwicklung gegen den DNA-Methylierungsinhibitor 2'-Desoxy-5-azacytidin zu verringern.

2. Bromodomäneninhibitor in Kombination mit dem DNA-Methylierungsinhibitor zur Verwendung nach Anspruch 1, wobei der Krebs ausgewählt ist aus Karzinomen, Sarkomen, Melanomen, Lymphomen und Leukämie.

## Revendications

1. (+)-JQ1, inhibiteur de la bromodomaine, en combinaison avec 2'-désoxy-5-azacytidine, inhibiteur de méthylation de l'ADN, pour utilisation dans le traitement du cancer par inhibiteur de méthylation de l'ADN pour surmonter la résistance et/ou pour réduire l'incidence de développer une résistance à l'inhibiteur de méthylation de l'ADN 2'-désoxy-5-azacytidine.

2. L'inhibiteur de bromodomaine en combinaison avec l'inhibiteur de méthylation de l'ADN pour utilisation selon la revendication 1, où le cancer est choisi parmi les carcinomes, les sarcomes, les mélanomes, les lymphomes et la leucémie.
